# EUROPEAN PATENT APPLICATION

(11) **EP 2 666 501 A1**
(43) Date of publication of application: **27.11.2013**
(21) Application number: 13169242.8
(22) Date of filing: 24.05.2013
(51) Int. Cl.: A61M 15/00

(54) **Inhaler having an inner body with air channel**

(30) Priority: 25.05.2012 TR 201206167; 08.02.2013 TR 201301562; 15.02.2013 TR 201301847; 19.02.2013 TR 201301950; 26.03.2013 TR 201303661
(71) Applicant: Arven Ilac Sanayi Ve Ticaret A.S., Istanbul 34460 (TR)
(72) Inventor: Toksöv, Zafer, 34460 Istanbul (TR); Cifter, ÜMIT, 34460 Istanbul (TR); Türkyilmaz, Ali, 34460 Istanbul (TR); Mutlu, Onur, 34460 Istanbul (TR)
(74) Representative: Sevinç, Erkan

(57) **Abstract**

The present invention relates to a dry power inhaler device wherein during administration of the said device (8), the air from the air hole (1) reaches the blister with the direction of the said air inlet channel (9), mixes with the powder medicament, carries the said medicament through the medicament channel within the mouthpiece (3) and administers to the user.

## Description

### Field of Invention

The present invention relates to a device for administering dry powder inhalation medicaments.

The present invention especially relates to improvements carried out in the part of dry powder inhaler devices enabling air flow.

### Prior Art

Diseases such as asthma, bronchitis, and COLD (Chronic Obstructive Lung Disease) substantially decrease the quality of human life, despite the developments which have been made in the diagnosis and therapy thereof in the recent years. It has been proposed to administer medicaments via inhalers for optimizing the treatment of such diseases. The inhaler route of treatment is the most preferred one and it is expected to remain so, as the first option, in the future. The most important advantage of using medicaments via inhalation is based on providing a more efficient therapy by making use of lower amounts of medicaments, delivering higher concentrations of medicaments to the airways, and particularly decreasing the systemic side effects of the medicaments. The most important causes of the lack of a satisfactory control of patients albeit the presence of quite efficient treatments against respiratory tract diseases are stated to be as the noncompliance, arising from the inefficient use of inhalers and from inadequate compliance to the physician-recommended treatments.

Nowadays, there have been developed various inhalation devices for administering inhalation medicaments. These devices are basically classified into two groups, i.e. metered dose inhalers and dry powder inhalers. These types of devices are structurally provided with basic components such as an actuator, counter, body, mouthpiece, cap, lock, etc. Additionally, powder inhalation medicaments are kept in carriers such as blisters, capsules, etc. Blisters are composed of two basic parts, a main layer provided with cavities carrying the medicament, and a strippable protective layer.

The users inhale the medicament with the help of the mouthpiece on the existing dry powder inhaler devices and by breathing, and the powder medicament reaches the target organ: lungs. For the blister or capsule inhalers, the medicament flowing after the blister is opened or the capsule is burst is directed to the mouthpiece and kept in an area connected to the mouthpiece. The mouthpiece is designed in a volume and length suitable for mouth anatomy. By this way, both administration of the medicament is facilitated and hygiene is provided. Inhaling the air into the lungs is an active action. With contraction of inspiration muscles, the frontal posterior diameter of chest cage widens and lengthens downwards. In accordance with Boyle-Marriotte Law, as volume of gas increases, its pressure decreases. According to this law, atmospheric air fills into the lungs since the pressure of air in the widening lungs shall be lower (than the pressure of atmospheric air). The air flow occurring with atmospheric air and inclining towards the body ensures that the powder medicament is taken without needing any other force. With the air flow, the powder in medicament is taken into body. Amount of the air inhaled during this process and how long it takes is very crucial.

In inhaler devices, an air circulation is initiated with breath of the user. The air passes through the air holes on outer body, and carries the medicament within the blister opened through the device. The powder medicament carried with air flow reaches the target area by passing through the channels within the mouthpiece. The critical issue at this point is that the air flow reaches the entire inner surface of the blister, and therefore, the medicament is administered to the user in an exact dose. This is achieved by applying the air passing through the bent on outer body into the stripped and ready blister in an ideal inclination and direction and without any interruption and obstacle. It is a necessity that the elements to be used to this end are placed, positioned correctly and designed to provide the most ideal air flow. Furthermore, diameters and relevant proportions of the channels and tanks through which the air passes and the medicament is carried are very important. These proportions are the factors which affect the speed of air flow and the convenient flow inclination. They play an important role in carrying the medicament without leaving any residue.

In inhaler embodiments explained in the applications numbered WO2010114506, WO2010114505 and WO2010114504, it is observed that there is a lack of structure which guarantees use of blister without any medicament residue.

In conclusion, in the field of inhaler devices, a novelty is required which shall operate with desired accuracy, and guarantee advantages of use and administration of the medicament in blister to the patient in an exact dose.

### Objectives and Brief Description of Invention

The present invention is an inhaler device used for dry powder inhalation and constructed to provide the air flow which shall carry the powder medicament in an exact dose, eliminating all problems mentioned above and bringing additional advantages to the relevant prior art.

Accordingly, the main objective of the present invention is to provide an inhaler device having an air inlet channel at a position in which the air flow shall not be prevented by the user during administration.

Another objective of the present invention is to provide an inhaler device operating with the desired accuracy and having optimum air flow limits.

Another objective of the present invention is to provide an inhaler device used for dry powder inhalation and constructed, with an air inlet channel, to provide the air flow which shall carry the powder medicament in an exact dose.

A further objective of the present invention is to provide an inhaler device with an air inlet channel enabling breathing of the user in target speed, continuity, rhythm and duration as well as administration of the medicament.

In order to achieve all objects described above and to emerge from the following detailed description, a dry powder inhaler device has been developed which comprises an outer body with at least one air hole, a mouthpiece housed in this outer body and an inner body with a channel for medicament within this mouthpiece, and a blister having cavities filled with the powder medicament.

In a preferred embodiment according to the present invention, at least one air inlet channel through which air free of medicament is passed, positioned between the air hole and medicament channel to enable the air inhaled during administration of the said device to sweep inner surface of cavity and prevent any medicament residue on the inner surface, and placed on the inner body independently from the said medicament channel, has been developed.

In another preferred embodiment according to the present invention, there is no surface between air hole and air hole channel which may change the direction of or prevent air flow.

In a further preferred embodiment according to the present invention, at least one part of the said air inlet channel hole is positioned opposite to at least one part of blister cavity.

In another preferred embodiment according to the present invention, the surface area of medicament channel is 1-25% bigger than the surface area of air inlet channel.

In a further preferred embodiment according to the present invention, the surface area of medicament channel is preferably 5-20% bigger than the surface area of air inlet channel.

In another preferred embodiment according to the present invention, the said air inlet channel also comprises a loophole.

In a further preferred embodiment according to the present invention, the said air inlet channel is positioned between air hole and blister cavity.

In another preferred embodiment according to the present invention, one or more air inlet channels are included preferably.

In a further preferred embodiment according to the present invention, during administration of the said device, the air from the air hole reaches the blister with the direction of the said air inlet channel, mixes with the powder medicament, carries the said medicament through the medicament channel within the mouthpiece and administers to the user.

Structural and characteristic properties and all advantages of the present invention shall be understood more clearly thanks to the figures below and detailed descriptions referring to these figures, and therefore, the evaluation should be made taking these figures and detailed descriptions into consideration.

### Brief Descriptions of Figures

Figure 1 is an illustration of a representative embodiment according to the present invention.
Figure 2 is an exemplary illustration of inhaler device body according to the present invention.

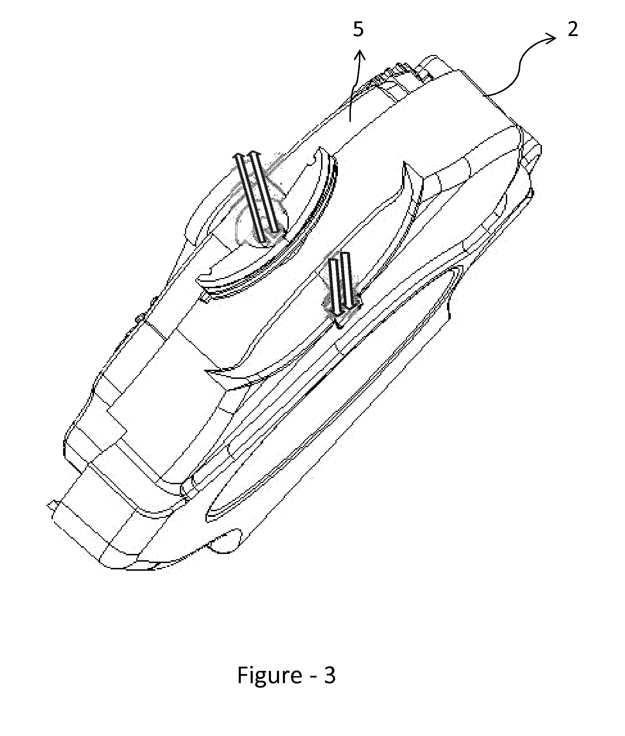

Figure 3 is an exemplary illustration of a part of device body and the mounted form of inner body according to the present invention.

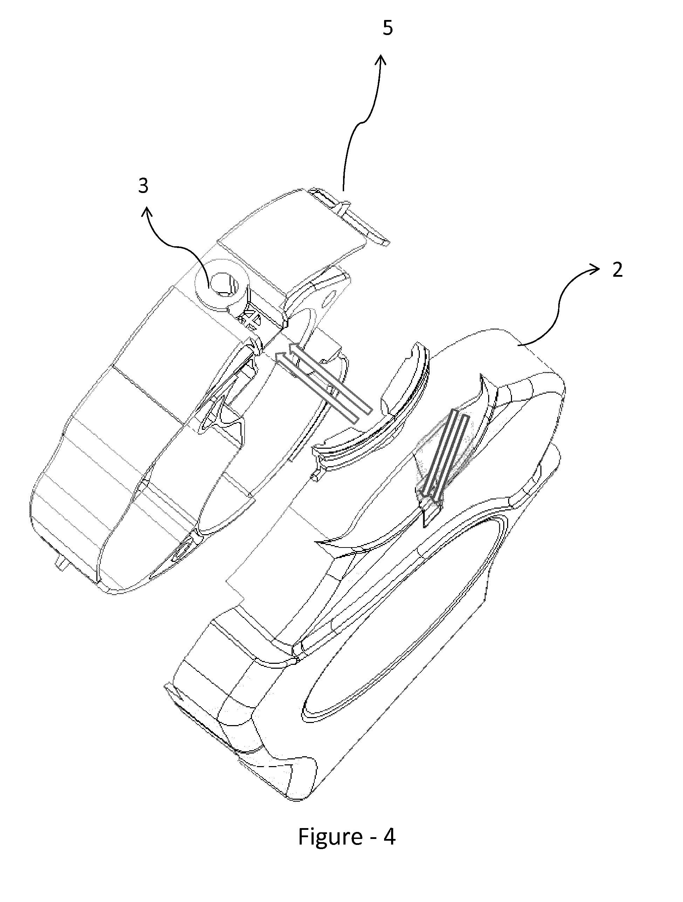

Figure 4 is an exemplary illustration of a part of device body and dismounted form of inner body according to the present invention.
Figure 5 is an exemplary illustration of a part of device body and mounted forms of inner body and blister according to the present invention.

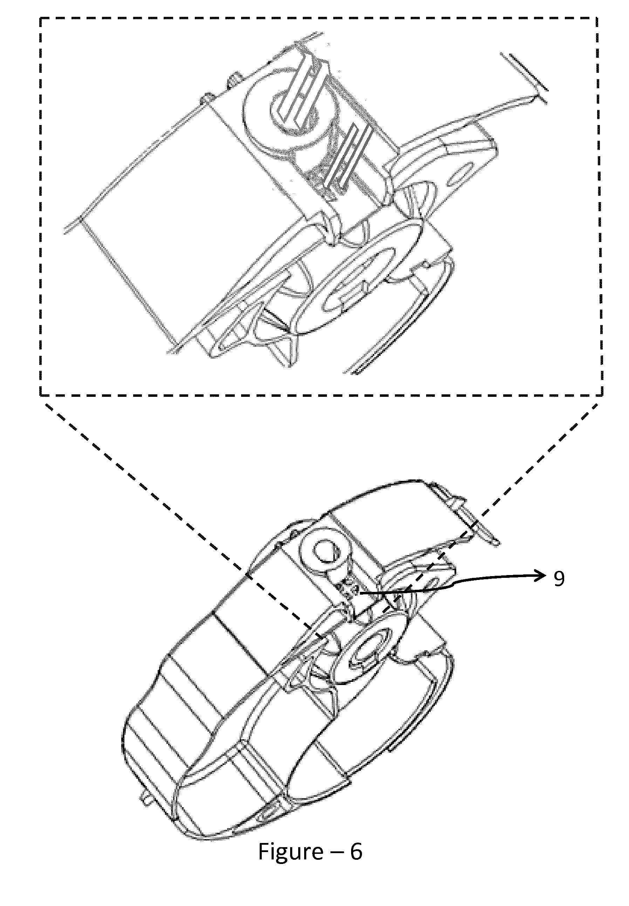

Figure 6 is an exemplary illustration of the inner body and air inlet channel according to the present invention.
Figure 7 is an exemplary illustration of the body, inner body and blister advancement mechanism according to the present invention.

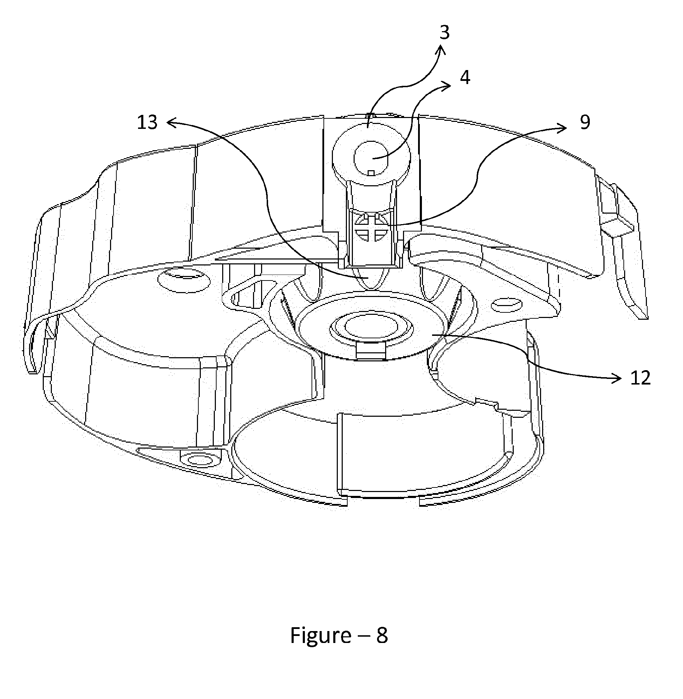

Figure 8 is an exemplary illustration of the inner body and air inlet channel according to the present invention.

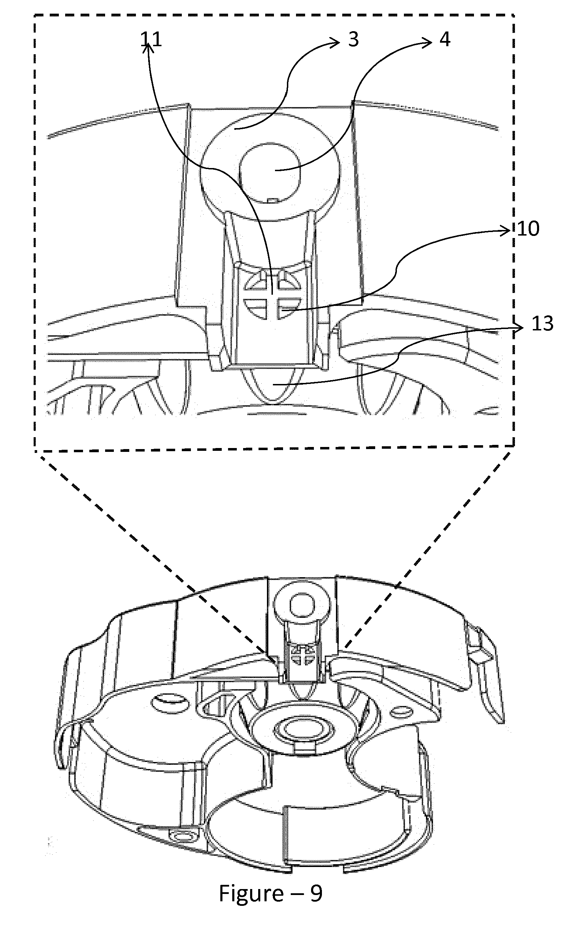

Figure 9 is an exemplary illustration of the inner body and air inlet channel according to the present invention.

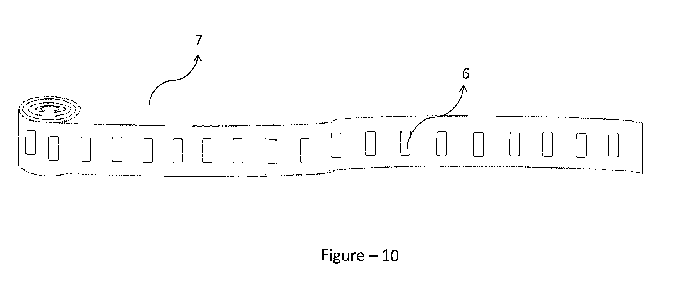

Figure 10 is an exemplary illustration of the blister according to the present invention.

### Reference Numbers in Figures

- 1.: Air hole
- 2.: Outer body
- 3.: Inner body Mouthpiece
- 4.: Medicament channel
- 5.: Inner Body
- 6.: Cavity
- 7.: Blister
- 8.: Inhaler device
- 9.: Air inlet channel
- 10.: Air inlet channel hole
- 11.: Loophole
- 12.: Guide gear
- 13: Guide gear slot

### Detailed Description of Invention

In the following detailed description, an inhaler device (8) according to the present invention shall be described illustratively by making references to the accompanying figures, only to make it clear without imposing any restrictions thereon.

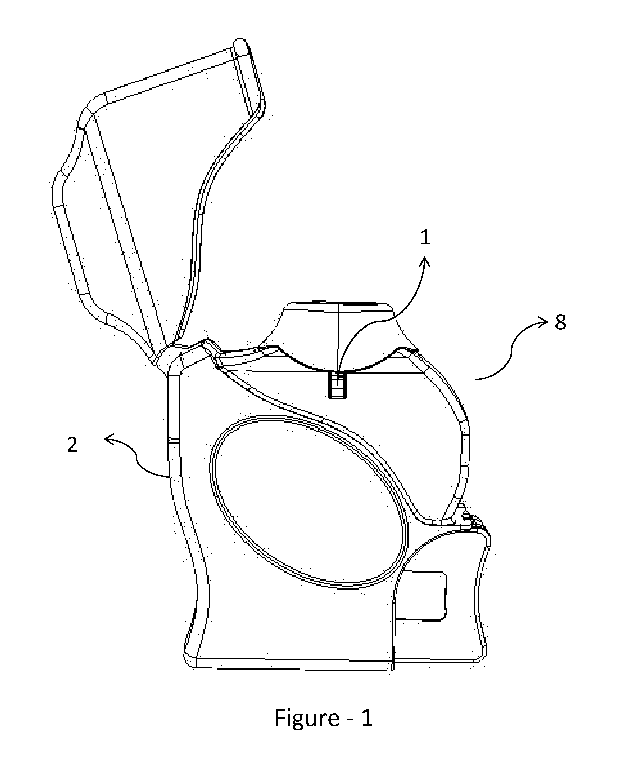

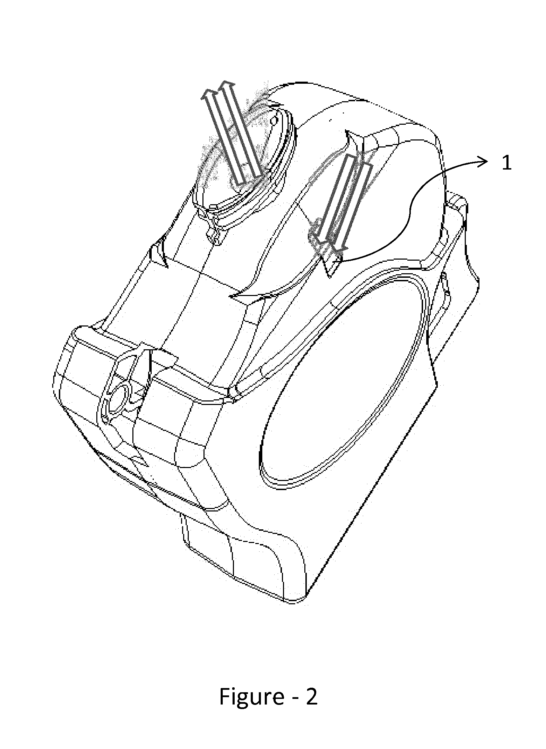

A device (8) according to the present invention, of which exemplary illustrations are given in Figure 1 and 2, is composed of an outer body (2) having an advancement mechanism for blisters with multiple cavities. There is a mouthpiece immediately upon this body.

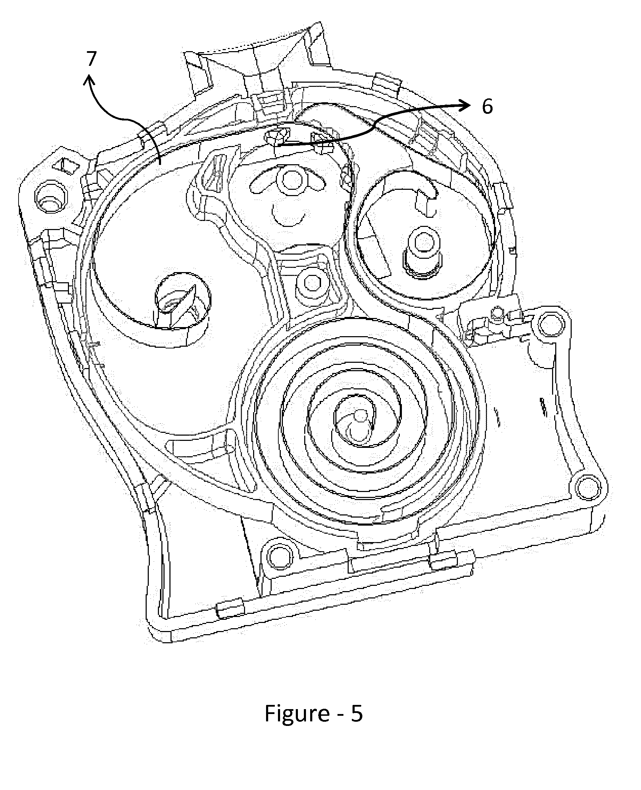

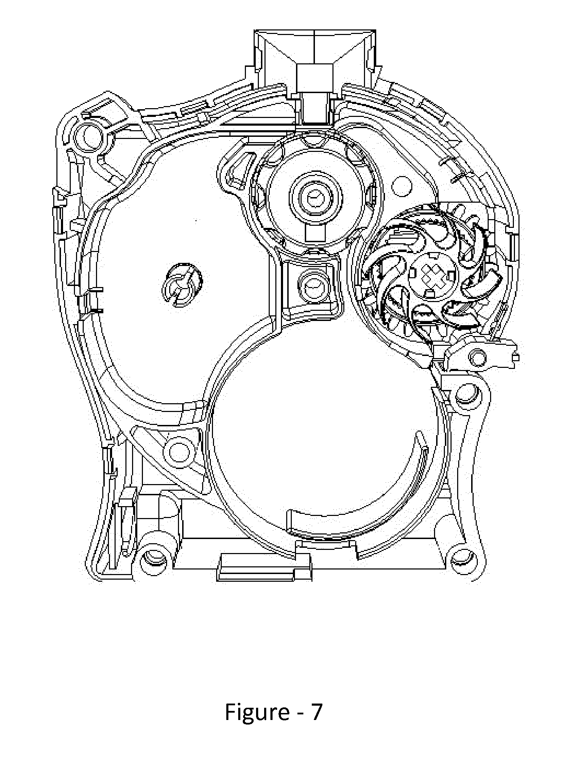

The device (8) body (2), of which exemplary illustrations are given in Figures 1, 2, 5, 7 8, 9, 10, have an air hole (1) thereupon. There is an inner body (5) housed within the body (2) and with a mouthpiece (3) and a medicament channel (4) constructed within this mouthpiece. There is an air inlet channel (9) immediately near the mouthpiece (3) of inner body and independent from this mouthpiece. The air inlet channel (9) is composed of a hole (10) and a loophole (11). A blister advancement mechanism gear set has been placed into inner part of outer body (2) in order to operate the inhaler. A blister (7) with cavities (6) full of powder medicament is positioned all along inter-channels of this gear set. In this gear set, immediately under the mouthpiece (3), there is a guide gear (12) into which blister (7) cavities (6) filled with powder medicament are placed through the slots (13) thereupon. Slots of guide gear (13) are positioned exactly under the medicament channel (4) in the mouthpiece (3). The air inlet channel (9) and the mouthpiece (3) are positioned side by side upon the inner body (5). The blister cavities (6) in the slots (13) are positioned under both of them (3, 9).

Under the light of detailed descriptions above, operation of the device according to the present invention is as follows. After the cap is opened, a force is applied to the device trigger by the user through grip surface. Then, the trigger body (2) is locked within the body (2). While the trigger (2) and the connected mechanism are operating, blister (7) passes through gear sets and gets stripped. The stripped blister cavity (6) is positioned to the medicament channel (4) in the medicament mouthpiece (3) and to the air inlet channel (9).

During the administration, with contraction of inspiration muscles of the user, the frontal posterior diameter of chest cage widens and lengthens downwards. Atmospheric air fills into the lungs since the pressure of air in the widening lungs shall be lower (than the pressure of atmospheric air). The air flow occurring with atmospheric air, inclining towards the body and passing through the air hole (1) passes through the air inlet channel (9) hole (10) and inclines directly towards the inner surface of open cavity (6) in the slot (13) of guide gear (12). This air flow ensures that the powder medicament is taken through the medicament channel (4) within mouthpiece (3) without needing another force. With the help of this air flow, the powder medicament in the device is completely taken into the body. The surface area of medicament channel is 1-25%, preferably 5 - 20 % bigger than the surface area of the said air inlet channel. The said channel surface areas are the parts out of the loopholes and enabling air flow. In this way, the ideal air flow speed and inclination are provided and the powder medicament in the blister is administered to the patient completely. Here, there is no set/obstacle in front of or on the air inlet channel which direct air flow to the blister, which may prevent, decrease or stop air flow. In this way, control of the direction of air flow is completely based on the air inlet channel (9). The loopholes prevent transit of big and foreign substances.

Thanks to the position of air inlet channel, inner surface of the cavity is swept completely by the air flow during administration, and therefore, it is ensured that the medicament is administered to the patient in an exact dose. The device operates with the desired accuracy and has optimum air flow limits. This is clearly understood with measurement results of NGI device.

Using inhaler devices with and without air channel, a "fine particle dose amount" analysis was performed. This analysis is made to determine fine particle amount reaching the lungs. The analysis was performed using Ph.Eur. 2.9.18 Apparatus E (Next Generation Impactor (NGI)). "Fine particle dose amount" analysis was performed with 4 kPa pressure drop stated in pharmacopoeia and the flow speed and duration required to take 4 L air.

| Results of fine particle dose amount **(Device without air channel)** | | |
|---|---|---|
| **Serial no** | **Salmeterol (mcg)** | **Fluticazon (mcg)** |
| 01 | 8,69 | 88,65 |
| 02 | 8,88 | 90,3 |
| 03 | 9,675 | 97,294 |
| 02 | 8,35 | 86,66 |
| 05 | 9,56 | 97,00 |
| 06 | 8,34 | 85,74 |

| Results of fine particle dose amount **(Device with air channel according to the present invention)** | | |
|---|---|---|
| 07 | 11,47 | 116,72 |
| 08 | 10,39 | 105,7 |
| 09 | 10,2 | 103,55 |
| 10 | 9,76 | 102,83 |
| 11 | 10,02 | 107,84 |
| 12 | 10,06 | 103,51 |

While 01 - 06 series show the values before the present invention, 06 - 12 series show the results obtained with the present invention. Under the light of these results, the change in fine particle dose amount to be taken by the patient using the present invention, and therefore, the efficiency thereof is observed explicitly at ideal air flow limits.

Additionally, the user breathes and uses the device at the desired speed, continuity, rhythm and duration.

Thanks to this embodiment developed, it is ensured that the user takes entire of effective dose of the medicament without needing to apply too much force at once.

In conclusion, thanks to the said embodiment, an inhaler device which is very accurate and operates safely has been developed.

Designs of components used for alternative embodiments vary based on the type of the device manufactured. As a result, protective scope of the present invention is stated in the claims attached, and it is certainly not limited to the mentioned in this detailed description for the purpose of examples. It is evident that an expert of the art can reveal the similar embodiments under the light of mentioned above without straying from main theme of the present invention.

## Claims

1. A dry powder inhaler device (8) comprising an outer body (2) with at least one air hole (1), a mouthpiece (3) housed in this outer body and an inner body (5) with a channel (4) for medicament within this mouthpiece, and a blister (7) having cavities (6) filled with the powder medicament, said device being **characterized by** comprising,
- at least one air inlet channel (9) through which air free of medicament is passed, positioned between the air hole (1) and medicament channel (4) to enable the air taken during administration of the said device (8) to sweep inner surface of cavity (6) and prevent any medicament residue on the inner surface, and placed on the inner body (5) independently from the said medicament channel (4).

2. The dry powder inhaler device (8) according to Claim 1, wherein there is no surface which may change the direction of or prevent the air flow between air hole (1) and air inlet channel (9).

3. The dry powder inhaler device (8) according to any of the preceding claims, wherein at least one part of hole (10) of the said air inlet channel (9) is positioned opposite to at least one part of blister (6) cavity (7).

4. The dry powder inhaler device (8) according to any of the preceding claims, wherein the surface area of medicament channel (4) is 1-25% bigger than the surface area of air inlet channel (9).

5. The dry powder inhaler device (8) according to any of the preceding claims, wherein the surface area of medicament channel (4) is preferably 5 - 20% bigger than the surface area of air inlet channel (9).

6. The dry powder inhaler device (8) according to any of the preceding claims, wherein the said air inlet channel (9) comprises a loophole (11).

7. The dry powder inhaler device (8) according to any of the preceding claims, wherein the said air inlet channel (9) is positioned between air hole (1) and blister cavity (6).

8. The dry powder inhaler device (8) according to any of the preceding claims, wherein the said air inlet channel (9) is one or more in number.

9. The dry powder inhaler device (8) according to any of the preceding claims, wherein during administration of the said device (8), the air from the air hole (1) reaches the blister with the direction of the said air inlet channel (9), mixes with the powder medicament, carries the said medicament through the medicament channel within the mouthpiece (3) and administers to the user.
